# EUROPÄISCHE PATENTANMELDUNG

(11) **EP 3 028 759 A1**
(43) Veröffentlichungstag der Anmeldung: **08.06.2016**
(21) Anmeldenummer: 15188752.8
(22) Anmeldetag: 07.10.2015
(51) Int. Cl.: B01D 53/14, C12M 1/00

(54) **VERFAHREN ZUR REINIGUNG VON BIOGAS UND BIOGASREINIGUNGSANLAGE**

(30) Priorität: 03.12.2014 DE 102014117748
(71) Anmelder: BMF HAASE Energietechnik GmbH, 24539 Neumünster (DE)
(72) Erfinder: KAHN,, Roland, 24598 Boostedt (DE)
(74) Vertreter: Dantz, Jan Henning

(57) **Zusammenfassung**

Bei einem Verfahren zur Reinigung von Biogas wird zunächst das Biogases zu einem Verdichter (6) zugeführt und dann auf einen Druck von mindestens 1,5 bar verdichtet. Dann werden wasserlösliche Bestandteile des Biogases in einer Waschvorrichtung (1) ausgewaschen, in dem das verdichtete Biogas durch einen Kontakt mit Wasser gereinigt wird. Anschließend kann dann das gereinigten Biogas abgeführt werden. Ferner wird eine Biogasreinigungsanlage bereitgestellt.

## Beschreibung

Die vorliegende Erfindung betrifft ein Verfahren zur Reinigung von Biogas und eine Biogasreinigungsanlage mit einer Waschvorrichtung, in der wasserlösliche Bestandteile des Biogases durch einen Kontakt mit Wasser zumindest teilweise ausgewaschen werden, wobei an der Waschvorrichtung eine Zufuhr für das Biogas und ein Auslass für das gereinigte Biogas und eine Wasserzufuhr und ein Wasserauslass vorgesehen sind. Die Reinigung des Biogases kann dabei vor oder in einer Biogasaufbereitungsanlage erfolgen.

In der DE 44 37 842 wird ein zweistufiger Biogaswäscher offenbart, der zwei übereinander angeordnete Waschzonen in einer Kolonne aufweist. Die untere und obere Waschzone sollen nacheinander vom Biogas angeströmt und dabei beregnet werden. Dadurch sollen gewisse Bestandteile in dem Biogas, wie organische Säuren, CO₂ oder Ammoniak, reduziert werden. Es ist vorgesehen Schwefelsäure und schwefelhaltige Säure in der oberen Waschzone auszuwaschen und Ammoniak in der unteren Waschzone auszuwaschen und dabei Bakterien unterschiedlicher Gattungen heranzuziehen. Das Waschwasser wird dabei zur Neutralisierung genutzt. Bei einem derartigen Biogaswaschverfahren ist es nachteilig, dass zum einen ein aufwendiger zweistufiger Prozess notwendig ist und zum anderen unterschiedliche Bakterien eingesetzt werden, für die ein bestimmtes Milieu notwendig ist.

Der Erfindung liegt daher die Aufgabe zugrunde, ein Verfahren zur Reinigung von Biogas und eine Biogasreinigungsanlage zu schaffen, die auf einfache Weise ein Biogas mit einer niedrigen Konzentration an Begleitstoffen, wie wasserlösliche Säuren oder Basen, gewinnt, insbesondere um nachfolgende Aggregate und Betriebsmittel zu schützen.

Diese Aufgabe wird durch ein Verfahren mit den Merkmalen des Anspruches 1 und eine Biogasreinigungsanlage mit dem Merkmal des Anspruches 8 gelöst.

Bei dem erfindungsgemäßen Verfahren wird das Biogas zu einem Verdichter zugeführt und dort wird das Biogas auf einen Druck von mindestens 0,5 bar verdichtet. Dann werden wasserlösliche Bestandteile des Biogases in einer Waschvorrichtung ausgewaschen, in der das verdichtete Biogas durch einen Kontakt mit Wasser kommt, um dann das gereinigte Biogas abzuführen. Es hat sich gezeigt, dass durch die Verdichtung des zu reinigenden Biogases die Reinigungsleistung erheblich verbessert ist, da die Abscheidung von wasserlöslichen Verbindungen durch die Verdichtung erhöht wird. Zudem ermöglicht die Verdichtung einen deutlich kompakteren Aufbau der Biogasreinigungsanlage.

Das Biogas kann vorzugsweise auf einen Überdruck von 0,5 bar bis 16 bar, bevorzugt auf 2-10 bar und besonders bevorzugt auf 5-8 bar verdichtet werden, bevor es mit dem Überdruck der Waschvorrichtung zugeführt wird.

Das Biogas erwärmt sich bei dem Verdichten. In einer bevorzugten Ausgestaltung des Verfahrens ist es daher vorgesehen, das Biogas nach dem Komprimieren auf eine Temperatur von 15-60° C, bevorzugt auf 20-50° C, und besonders bevorzugt auf 30-40° C abzukühlen. Für die Abkühlung dient ein Wärmetauscher. Es ist auch möglich, das Biogas in zwei oder mehr hintereinander geschalteten Verdichtern zu komprimieren oder den Wärmetauscher für die Abkühlung des Biogases zwischen zwei Verdichtern vorzusehen. Nach dem Verdichten und Abkühlen des Biogases kann dieses der Waschvorrichtung zugeführt werden.

Gemäß einer weiteren Ausgestaltung der Erfindung beträgt eine Verweilzeit des verdichteten Biogases in der Waschvorrichtung mindestens 1 Sekunde, bevorzugt 2-10 Sekunden, und besonders bevorzugt 4-6 Sekunden. Für eine gute Reinigungsleistung kann dabei die relative Feuchte des Biogases schon vor der Waschvorrichtung 100 % betragen. Der Volumenstrom des Biogases beträgt beispielsweise zwischen 100 und 4000 Nm³/h, vorzugsweise 500 und 2000 Nm³/h.

Die Waschvorrichtung kann dabei das verdichtete Biogas mit Wasser über mindestens eine Einspritzdüse besprühen, so dass Wasser als fein verteilte Wassertröpfchen in Kontakt mit dem Biogas kommt. Dadurch wird eine aktive Tröpfchenzone gebildet, in der die Wassertröpfchen im Gegenstrom zum Gas von oben nach unten gesprüht werden. Oberhalb der aktiven Tröpfchenzone, kann das gereinigte Biogas abgezogen werden, ggfs. nach vorheriger Entfernung von Wassertröpfchen über einen Abscheider. Es ist auch möglich, die Waschvorrichtung im Gleichstrom, so dass Wassertröpfchen und das Biogas in dieselbe Richtung strömen, oder im Kreuzstrom, also mit sich kreuzenden Strömungswegen, zu betreiben.

Wenn die Waschvorrichtung eine Einspritzdüse umfasst, die fein verteilte Wassertröpfchen versprüht, kann eine unterschiedliche Löslichkeit der Biogaskomponenten in Wasser ausgenutzt werden. Es ist dabei wünschenswert mehrere Biogaskomponenten in den Wassertröpfchen zu lösen. Vorzugsweise verbleibt Methan in einer hohen Konzentration in dem Gas gewonnen und restliche wasserlösliche Bestandteile verbleiben in den Wassertröpfchen. Durch die fein verteilten Wassertröpfchen wird eine große Kontaktfläche zwischen dem komprimierten Biogas und dem Wasser erhalten. Es ist daher bevorzugt vorgesehen, dass eine Kontaktfläche zwischen dem komprimierten und abgekühlten Biogas und den fein verteilten Wassertröpfchen groß ist. Dies kann durch ein Ausbilden möglichst kleiner Wassertröpfchen möglich sein und/oder durch Erhöhen einer Anzahl der Einspritzdüsen und/oder durch Vergrößerung Waschkolonne. Dabei erhöht sich die spezifische Oberflächengröße der Wassertröpfchen mit einer abnehmenden Größe der Wassertröpfchen.

Alternativ kann das zu reinigende Biogas auch durch das Wasser durchgeleitet werden, so dass feine Bläschen erzeugt werden, die durch das umgebende Wasser gereinigt werden.

Es ist vorzugsweise vorgesehen, dass das gereinigte Biogas, das im Wesentlichen frei von wasserlöslichen Bestandteilen ist, an einem oberen Bereich eines Behälters abgeführt wird. Dabei kann ein Demisterabscheider vorgesehen sein, um im Wesentlichen letzte Wassertröpfchen aus dem gereinigten Biogas zu entfernen. Das Biogas weist nach der Reinigung 40 bis 70% Methan auf. Das gereinigte Biogas kann dann nach einer nachfolgenden CO₂-Entfernung eine höhere Methankonzentration, beispielsweise in einem Bereich von 96-99 Vol.-% enthalten. Weitere nach der Reinigung des Biogases durchführbare Verfahren zur Aufbereitung von Biogas sind insbesondere die Genosorbwäsche, Wasserwäsche, Membrantrennverfahren und pressure swing adsorption (PSA).

Eine weitere Ausgestaltung der Erfindung sieht vor, dass das bei dem Abkühlen des verdichteten Biogases mittels des Wärmetauschers entstehende Kondensat als Flüssigkeit zum Auswaschen der wasserlöslichen Bestandteile des Biogases eingesetzt wird. Dabei kann das Kondensat in einem Vorlagebehälter aufgefangen werden, um dieses der Waschvorrichtung zuzuführen. Die Zuführung des Kondensates kann dabei entweder kontinuierlich oder geregelt nach Bedarf erfolgen.

Vorzugsweise wird das mit wasserlöslichen Bestandteilen beladene Wasser im unteren Bereich des Behälters an einem Sumpf abgezogen und einem Ausgasraum zum zumindest teilweise Ausgasen der gelösten Bestandteile zugeführt. Unter anderem kann dort das gelöste CO₂ und/oder Methan ausgasen.

Die wasserlöslichen Bestandteile des Biogases sind organische Säuren, wie Ameisensäure, Essigsäure oder Propionsäure und/oder anorganische Säuren, wie Schwefelwasserstoff oder Schwefelsäure und/oder weitere Gasbestandteile, wie CO₂ oder Methan und/oder basische Komponenten, wie Ammoniak. Die Zusammensetzung variiert je nach Biogassubstrateinsatz. Beispielsweise können landwirtschaftliche Rohstoffe, wie Mais, Getreide oder getrocknetes Gras als Biogassubstrat dienen. Ferner können organische Abfälle aus Haushalten und Grünflächen, oder industrielle organische Abfälle eingesetzt werden. Auch Deponiegas ist eine Form eines Biogases, das mit der Erfindung gereinigt werden kann.

Die Waschvorrichtung weist einen pH-Messer auf, mittels dem der pH-Wert des aus dem Wasserauslass abgeführten Wassers messbar ist. Das abgeführte Wasser kann dann zirkulierend wieder der Waschvorrichtung zugeführt werden, während der pH-Wert erfasst wird. Bei einem unerwünscht niedrigem pH-Wert aufgrund in dem Wasser gelöster Säuren, wird das Wasser abgeführt und durch sauberes Wasser ersetzt. Dadurch kann Wasser in mehreren Zyklen für das Auswaschen der löslichen Bestandteile verwendet werden.

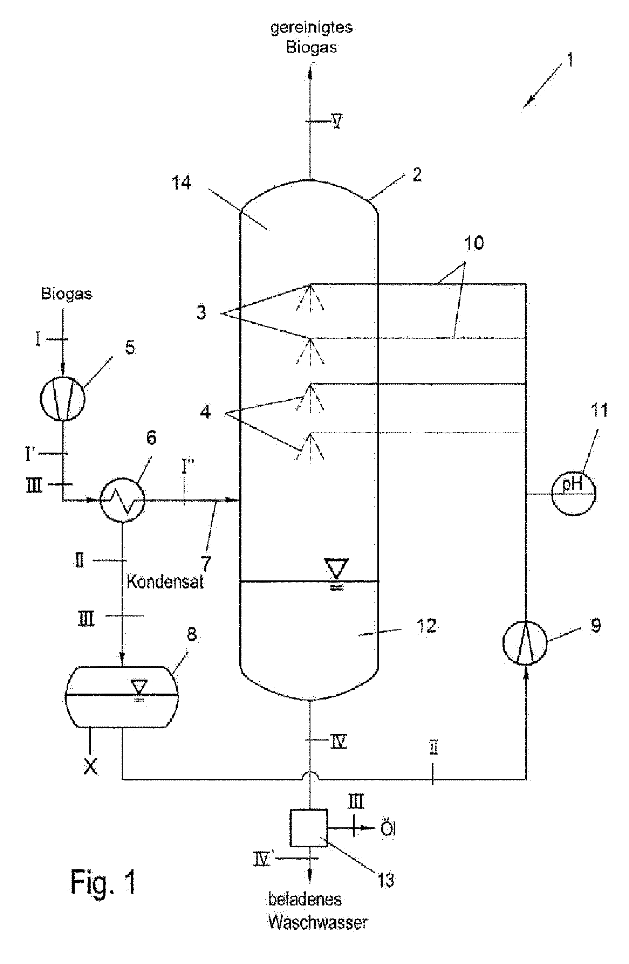

Ein Ausführungsbeispiel der Erfindung ist in Figur 1 dargestellt und nachfolgend näher erläutert. Es zeigt:
- Figur 1: eine schematische Darstellung einer Biogasreinigungsanlage.

Die in Figur 1 gezeigte Biogasreinigungsanlage zum Auswaschen wasserlöslicher Bestandteile eines Roh-Biogases I beispielsweise für eine Verwendung im Erdgasnetz umfasst eine Waschvorrichtung 1 mit einem Behälter 2. In den Behälter 2 wird zu reinigendes Biogas eingespeist. Über Einspritzdüsen 3 werden fein verteilte Wassertröpfchen 4 zum Auswaschen wasserlöslicher Bestandteile des Biogases versprüht. Es ist möglich eine oder mehrere Einspritzdüsen 3 vorzusehen, die auch unterschiedlich große Wassertröpfchen aussprühen können.

Das Biogas I wird vor dem Einspeisen in den Behälter 2 in einem Verdichter 5 auf einen Überdruck komprimiert, beispielsweise auf über 0,5bar. Der Druck nach dem Verdichter 5 kann in einem Bereich zwischen 2 bis 10 bar, insbesondere zwischen 5 bis 8 bar liegen. Bei dem Verdichten erwärmt sich das zu reinigende Biogas I und wird daher anschließend über einen Wärmetauscher 6 gekühlt. Vorteilhafterweise beträgt die Temperatur des abgekühlten Biogases I" zwischen 15-60° C, bevorzugt 20-50° C, besonders bevorzugt 30-40° C. Das Komprimieren und/oder Abkühlen kann dabei auch in mehreren Stufen erfolgen. Auch kann zuerst ein Verdichter, dann ein Wärmetauscher, gefolgt von einem weiteren Verdichter und Wärmetauscher vorgesehen sein.

Das komprimierte und abgekühlte Biogas I" wird dann über eine Leitung 7 in den Behälter 2 unterhalb beim Gegenstrom oder oberhalb beim Gleichstrom der Einspritzdüsen 3 eingespeist. Die feinen Wassertröpfchen 4 aus den Einspritzdüsen 3 strömen im Gegenstrom zum verdichteten Biogas. Eine Verweilzeit des zu reinigenden Biogases in einer aktiven Tröpfchenzone in dem Behälter 2 ist so gewählt, dass diese mindestens 1 Sekunde, bevorzugt 2-10 Sekunden, und besonders bevorzugt 4-6 Sekunden beträgt. Die aktive Tröpfchenzone beschreibt dabei einen Bereich in dem Behälter 2, in dem die Wassertröpfchen 4 im Gegenstrom zum verdichteten und abgekühlten Biogas I" geführt sind. Die relative Feuchte in dem Behälter 2 kann dabei 100% betragen. Die Absorption der wasserlöslichen Bestandteile ist durch die Verdichtung und Abkühlung des Biogases I" in dem Waschwasser erhöht.

Als Waschwasser kann zumindest teilweise das bei dem Abkühlen des verdichteten Biogases I' mittels des Wärmetauschers 6 entstehende Kondensat II eingesetzt werden. Das Kondensat II wird hierfür in einem Vorlagebehälter 8 aufgefangen. Dann kann das Kondensat II aus dem Vorlagebehälter 8 z.B. mittels einer Pumpe 9 über Zulaufleitungen 10 zu den Einspritzdüsen 3 gepumpt werden, wo es unter Überwindung des Gegendruckes in dem Behälter 2 versprüht wird.

Überschüssiges Kondensat und/oder Öltröpfchen III kann aus dem Vorlagebehälter 8 über ein Ventil (X) abgelassen werden.

Ein mit wasserlöslichen Bestandteilen des verdichteten und abgekühlten Biogases I" beladenes Wasser IV ist an einem Sumpf 12 des Behälters 2 abgeführt und kann an einem Ausgasraum zum zumindest teilweise Ausgasen der gelösten Bestandteile zugeführt sein. Es können bei dem Verdichten des zu reinigenden Biogases durch den Verdichter 5 Öltröpfchen III zugeführt werden, die dann im Vorlagebehälter 12 wieder abgetrennt werden. Das beladene Wasser IV' wird dann dem Ausgasraum zugeführt, wo zumindest gelöstes CO₂ und Methan ausgast. Es bleiben unter Anderem wasserlösliche organische Säuren im Kondensat gelöst.

Das Waschwasser zirkuliert um es mehrfach in dem Behälter 2 einzusetzen. Zur Messung des pH-Wertes des Waschwassers ist eine pH-Sonde 11 vorgesehen. Sollte der pH-Wert außerhalb eines vorbestimmten Bereiches liegen, kann dann frisches Waschwasser oder neues Kondensat zugeführt werden. Hierfür kann die pH-sonde 11 mit einer entsprechenden Steuerung der Anlage verbunden sein. Optional kann auch auf eine pH-Sonde 11 verzichtet werden, beispielsweise wenn kontinuierlich Waschwasser als Kondensat oder als Frischwasser zugeführt wird. Es kann bei Bedarf auch über eine Steuerung Frischwasser zu dem Behälter 2 zugegeben werden.

Es ist ferner möglich die Löslichkeit wasserlöslicher Bestandteile des verdichteten und abgekühlten Biogases I" in dem Waschwasser weiter zu erhöhen, indem eine Temperatur des Waschwassers optimiert ist. Die Waschwassertemperatur beträgt vorteilhaft 5-50° C, bevorzugt 10-40° C, und besonders bevorzugt 20-35° C.

In dem zu reinigenden Biogas können als wasserlösliche Bestandteile organische Säuren, wie Ameisensäure, Essigsäure oder Propionsäure und/oder anorganische Säuren, wie Schwefelsäure oder Schwefelwasserstoff vorkommen. Ferner kann das Biogas neben CO₂ und Methan weitere Gasbestandteile, Ammoniak, umfassen.

In dem Behälter 2 wird das gereinigte Biogas V, das im Wesentlichen frei von wasserlöslichen Bestandteilen ist abgeführt. Vorteilhafterweise können letzte Wassertröpfchen 4 aus dem gereinigten Biogas V durch einen Demisterabscheider an dem Behälter 2 (nicht dargestellt) entfernt werden.

Das gewonnene gereinigte Biogas V ist im Wesentlichen frei von wasserlöslichen Säuren und Basen. Die Methankonzentration des eingesetzten Roh-Biogases I liegt im Wesentlichen zwischen 35-70 Vol.-%.

In der dargestellten Biogasreinigungsanlage 1 wird das Wasser über Düsen 3 eingesprüht. Es ist auch möglich, das zu reinigende Biogas in ein Waschwasser einzuleiten, wie beispielsweise bei Blasensäulen. Dann kann das zu reinigende und verdichtete Biogas in Form von feinen Bläschen durch einen Behälter aufsteigen und dadurch gereinigt werden.

## Patentansprüche

1. Verfahren zur Reinigung von Biogas, mit den folgenden Schritten:
- Zuführen des Biogases zu einem Verdichter (5);
- Verdichten des Biogases auf einen Druck von mindestens 1,5 bar;
- Auswaschen wasserlöslicher Bestandteile des Biogases in einer Waschvorrichtung (1), in der das verdichtete Biogas durch einen Kontakt mit Wasser gereinigt wird, und
- Abführen des gereinigten Biogases.

2. Verfahren nach Anspruch 1, **dadurch gekennzeichnet, dass** das Biogas auf einen Überdruck von 0,5 bar bis 16 bar, bevorzugt auf 2 bis 10 bar, und besonders bevorzugt auf 5 bis 8 bar, verdichtet wird.

3. Verfahren nach Anspruch 1 oder 2, **dadurch gekennzeichnet, dass** das Biogas nach dem Verdichten auf eine Temperatur von 15 bis 60° C, bevorzugt auf 20 bis 50° C, und besonderes bevorzugt auf 30 bis 40° C abgekühlt wird.

4. Verfahren nach einem der vorhergehenden Ansprüche, **dadurch gekennzeichnet, dass** eine Verweilzeit des verdichteten Biogases in der Waschvorrichtung (1) mindestens 1 Sekunden, bevorzugt 2 bis 10 Sekunden, und besonders bevorzugt 3 bis 6 Sekunden, beträgt.

5. Verfahren nach einem der vorhergehenden Ansprüche, **dadurch gekennzeichnet, dass** in der Waschvorrichtung (1) das verdichtete Biogas mit Wasser in Kontakt kommt, das über mindestens eine Einspritzdüse (3) als fein verteilte Wassertröpfchen (4) versprüht wird.

6. Verfahren nach einem der vorhergehenden Ansprüche, **dadurch gekennzeichnet, dass** die Waschvorrichtung (1) hinsichtlich der Wasserzufuhr und des Wasserauslasses und der Strömung des Biogases im Gegenstrom betrieben wird.

7. Verfahren nach einem der vorhergehenden Ansprüche, **dadurch gekennzeichnet, dass** eine relative Feuchte des Biogases vor der Waschvorrichtung etwa 100 % beträgt.

8. Verfahren nach einem der vorhergehenden Ansprüche, **dadurch gekennzeichnet, dass** der Volumenstrom des Biogases zwischen 100 und 4000 Nm³/h beträgt.

9. Biogasreinigungsanlage, insbesondere zur Durchführung des Verfahrens nach einem der Ansprüche 1 bis 8, mit einer Waschvorrichtung (1), in der wasserlösliche Bestandteile des Biogases durch einen Kontakt mit Wasser zumindest teilweise ausgewaschen werden, wobei an der Waschvorrichtung eine Zufuhr für das Biogas und ein Auslass für das gereinigte Biogas und eine Wasserzufuhr und ein Wasserauslass vorgesehen sind, **dadurch gekennzeichnet, dass** vor der Waschvorrichtung (1) ein Verdichter (5) angeordnet ist, mittels dem das zu reinigende Biogas mit Überdruck zu der Waschvorrichtung (1) zuführbar ist und die Waschvorrichtung (1) mit Überdruck betreibbar ist.

10. Biogasreinigungsanlage nach Anspruch 9, **dadurch gekennzeichnet, dass** zwischen der Waschvorrichtung (1) und dem Verdichter (5) ein Wärmetauscher (6) zur Kühlung des verdichteten Biogases vorgesehen ist.

11. Biogasreinigungsanlage nach Anspruch 9 oder 10, **dadurch gekennzeichnet, dass** das Wasser zum Reinigen des Biogases über eine Pumpe (9) der Waschvorrichtung (1) mit Überdruck zuführbar ist.

12. Biogasreinigungsanlage nach Anspruch 10 oder 11, **dadurch gekennzeichnet, dass** das bei dem Abkühlen des verdichteten Biogases an dem Wärmetauscher (6) entstehende Kondensat der Waschvorrichtung (1) zum Auswaschen der wasserlöslichen Bestandteile des Biogases zuführbar ist.

13. Biogasreinigungsanlage nach einem der Ansprüche 9 bis 12, **dadurch gekennzeichnet, dass** die Waschvorrichtung (1) einen Behälter (2) umfasst, in dem mehrere Düsen (3) zum Versprühen von unter Druck stehendem Wasser vorgesehen sind.

14. Biogasreinigungsanlage nach Anspruch 13, **dadurch gekennzeichnet, dass** in einem unteren Bereich des Behälters (2) der Wasserauslass für das beladene Wasser vorgesehen ist.

15. Biogasreinigungsanlage nach einem der Ansprüche 9 bis 14, **dadurch gekennzeichnet, dass** die Waschvorrichtung (1) einen pH-Messer (11) aufweist, mittels dem der pH-Wert des aus dem Wasserauslass abgeführten Wassers messbar ist.

16. Biogasreinigungsanlage nach einem der Ansprüche 9 bis 15, **dadurch gekennzeichnet, dass** der Biogasreinigungsanlage ein Verfahren zur CO2-Abscheidung nachgeschaltet wird, das den Druck des verdichteten Biogases weiter nutzt.
